# FASCICULE DE BREVET EUROPEEN

(11) **EP 0 949 165 B1**
(45) Date de publication et mention de la délivrance du brevet: **06.08.2003**
(21) Numéro de dépôt: 98201059.7
(22) Date de dépôt: 06.04.1998
(51) Int. Cl.: B65G 33/04

(54) **Dispositif de transport d'objets**
Vorrichtung zum Fördern von Gegenständen
Device for conveying objects

(43) Date de publication de la demande: 13.10.1999
(73) Titulaire: Sybermat, 1348 Louvain-la-Neuve (BE)
(72) Inventeur: Aelgoet, Arthur, 1400 Nivelles (BE)
(74) Mandataire: Claeys, Pierre

(56) Documents cités:
- EP-A- 0 645 093
- DE-A- 3 819 093
- GB-A- 2 104 029
- GB-A- 2 290 221

## Description

La présente invention concerne un dispositif de transport d'objets selon une direction et un sens déterminés, comportant une vis de transport, dont le creux entre filets successifs est adapté pour entrer en prise avec une portion déterminée des objets à transporter, et comportant un guide agencé sensiblement parallèlement à l'axe de la vis de manière à maintenir dans le creux de la vis ladite portion des objets à transporter.

Les dispositifs de transport à vis connus à ce jour ne prévoient, à la connaissance de la déposante, qu'une fonction de transport de la part de la vis. D'autres fonctions, comme un positionnement particulier de l'objet comme dans GB-A-2 290 221, ou une rotation de celui-ci sur lui-même ou d'une portion de l'objet par rapport à une autre portion de celui-ci, en vue d'un positionnement ou orientation relatif de l'une par rapport à l'autre, au cours du transport doivent présentement être accomplies par d'autres organes agencés dans le dispositif de transport.

Cet état de fait non seulement augmente très sensiblement le coût des dispositifs de transport connus du genre ci-dessus mais surtout accroît la difficulté de les aménager en fonction des objets à transporter. C'est le cas par exemple lorsque ces dispositifs sont destinés à transporter à un moment un lot d'objets d'un type et forme, éventuellement avec un type de fonction(s) particulière(s) à accomplir au cours du transport et à un autre moment un autre lot d'objets d'un autre type et forme, éventuellement avec un autre type de fonction(s) particulière(s) à accomplir au cours du transport.

La présente invention a pour but de remédier aux inconvénients cités ci-dessus, et à d'autres connus de l'homme de métier mais non mentionnés ici, et de procurer un dispositif de transport à vis dans lequel la vis de transport puisse remplir non seulement sa fonction connue mais aussi une ou des autres, comme celle d'un positionnement ou orientation particulier de l'objet, ou d'une portion de celui-ci par rapport à une autre, au cours du transport.

A cet effet, suivant l'invention, le dispositif de transport précité comporte de plus, sur au moins une partie de filet de la vis, des moyens agencés pour agir sur au moins une autre portion des objets au cours de leur transport.

Suivant une forme de réalisation, les moyens agencés pour agir sur l'autre portion des objets au cours de leur transport sont situés au sommet de ladite partie de filet. Avantageusement, les moyens agencés pour agir sur l'autre portion des objets comportent au moins une excroissance agencée pour entrer temporairement en prise avec cette autre portion.

Suivant une forme de réalisation particulière de l'invention, sur l'excroissance susdite est prévue au moins localement une excroissance supplémentaire agencée pour entrer en prise, au moins temporairement, au cours du transport avec une autre portion de l'objet, différente de celles précitées.

Avantageusement, l'excroissance susdite ou/et l'excroissance supplémentaire est/sont au moins une tige ou lame, de préférence souple, dressée au sommet d'un filet de la vis ou respectivement d'une excroissance.

Suivant une forme de réalisation préférée de l'invention, l'excroissance agencée sur le sommet du filet de vis et/ou l'excroissance supplémentaire précitée forme(nt) au moins un segment de filet de vis, ou un filet complet, dont le pas peut être différent de celui de la vis proprement dite.

D'autres détails et particularités de l'invention ressortiront des revendications secondaires et de la description des dessins qui sont annexés au présent mémoire et qui illustrent, à titre d'exemples non limitatifs, des formes de réalisation particulières du dispositif de transport de l'invention.

La figure 1 représente schématiquement, en coupe transversale, des éléments à décrire du dispositif de transport de l'invention ainsi qu'un objet, pris lui aussi à titre d'exemple, en cours de transport.

La figure 2 représente schématiquement l'objet de la figure 1, choisi à titre d'exemple, projeté sur un plan parallèle à l'axe de celui-ci et perpendiculaire au plan de coupe de la figure 1.

La figure 3 représente schématiquement, en perspective avec brisures, une vis d'un modèle pour le dispositif suivant l'invention.

La figure 4 représente schématiquement, en perspective avec brisures, une vis d'un autre modèle pour le dispositif suivant l'invention.

Les figures 5 à 7 représentent en section axiale, avec brisures, trois types de vis qui peuvent être utilisées dans le dispositif de l'invention.

Dans les différentes figures, les mêmes références désignent des éléments analogues ou semblables.

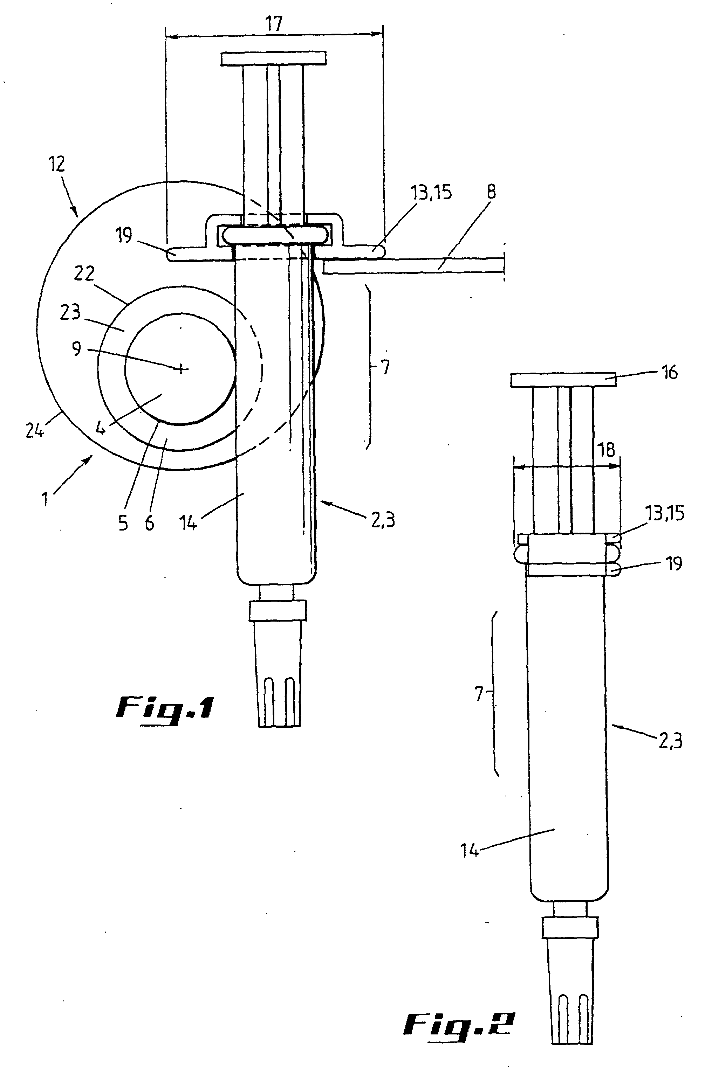

Le dispositif 1 (figure 1) de l'invention est destiné à transporter des objets 2, comme des seringues 3, selon une direction et un sens déterminés. A cet effet, le dispositif 1 comporte une vis de transport 4, connue en soi, dont le creux 5 entre filets 6 successifs est adapté pour entrer en prise avec une portion déterminée 7 des objets 2 à transporter. Le dispositif 1 comporte de plus un guide 8 agencé sensiblement parallèlement à l'axe 9 de la vis 4 de manière à maintenir dans le creux 5 de la vis 4 ladite portion déterminée 7 des objets 2 à transporter.

Suivant l'invention, la vis de transport 4 comporte de plus, sur au moins une partie de filet 6 de la vis 4, des moyens 12 agencés pour agir sur au moins une autre portion 13 desdits objets 2 au cours de leur transport.

Ainsi, pour l'exemple des seringues 3, celles-ci peuvent présenter principalement, comme portion déterminée 7, leur corps cylindrique 14, sur lequel peuvent agir les filets 6 et creux 5 de la vis 4, et, comme autre portion 13, leur collet 15 destiné entre autre à l'appui des doigts lors de la manoeuvre de leur piston 16. Ce collet 15 peut présenter, dans un plan transversal à l'axe du corps cylindrique 14, une forme oblongue comme le fait percevoir la différence entre une dimension 17 de ce collet 15 à la figure 1 et l'autre dimension 18 du même à la figure 2. Dans le cas décrit, les moyens 12 peuvent être agencés pour agir sur les bords 19 de ce collet 15 dans le but de le positionner ou plus précisément de l'orienter, en fonction de sa forme oblongue, de manière à ce que sa plus grande dimension 17 soit orientée sensiblement perpendiculairement à l'axe 9 de la vis de transport 4, comme cela peut être souhaité par exemple lors de la manipulation automatisée de seringues 3 pour leur emballage.

Comme le montrent les figures, les moyens 12 agencés pour agir sur au moins une autre portion 13 des objets 2 au cours de leur transport sont avantageusement situés au sommet 22 de ladite partie de filet 6. Cela n'exclut pas que ces moyens 12 pourraient être disposés ou fixés au moins en partie sur un ou les flancs 23 des filets 6, et de préférence alors près dudit sommet 22.

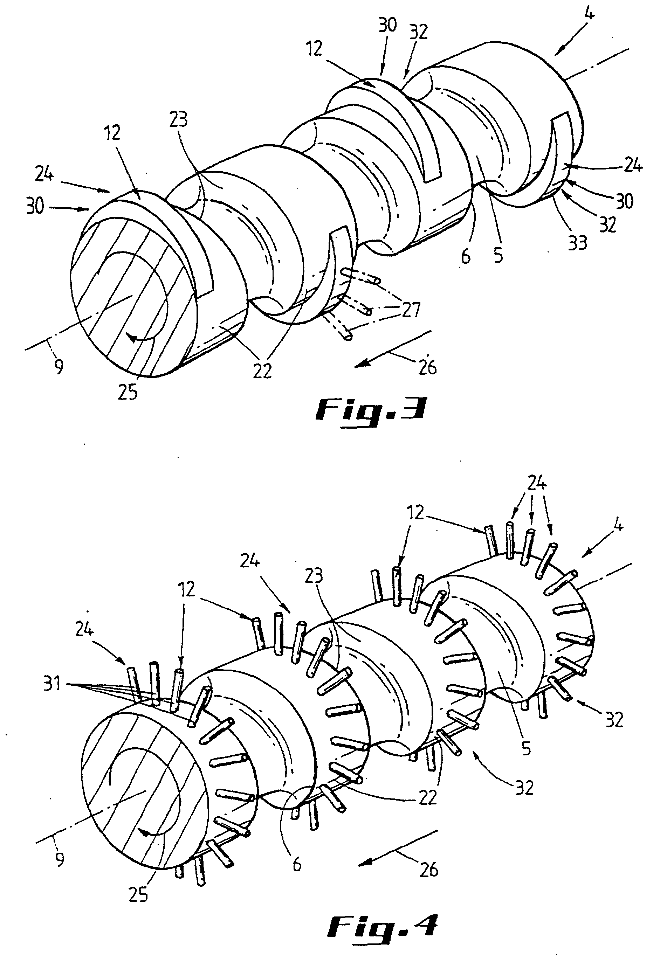

De manière particulière, les moyens 12 agencés pour agir sur l'autre portion 13 des objets 2 au cours de leur transport peuvent comporter, ou être constitués par, au moins une excroissance 24 (figure 3 ou 4) agencée pour entrer en prise temporairement, au cours du transport, avec l'autre portion 13 de l'objet 2 considéré. L'homme de métier comprend l'action qu'aura l'excroissance 24 ou qu'auront plusieurs excroissances 24 successives sur le collet 15 de la ou des seringues 3, citées à titre d'exemple en tant qu'objet 2 à transporter et à orienter ou positionner, au cours de la rotation de la vis de transport 4 suivant la flèche 25 pour faire avancer lesdits objets 2 dans le sens de la flèche 26.

Comme le montre la figure 3, il peut encore être prévu sur l'excroissance 24 susdite, au moins localement, une ou plusieurs excroissances supplémentaires 27 agencées pour entrer en prise, au moins temporairement, au cours du transport avec une autre ou troisième portion (non représentée) de l'objet 2 à transporter, différente de celles précitées 7 et 24. On peut imaginer par exemple que l'objet 2 présente, à un niveau correspondant aux excroissances supplémentaires 27, ladite troisième portion et que celle-ci est à même de tourner sur elle-même par rapport à l'autre ou seconde portion 13 précitée autour d'un axe de l'objet 2 qui est usuellement vertical et perpendiculaire à l'axe 9 en cours de transport. Dans ce cas, ladite troisième portion peut à son tour être orientée de son côté par les excroissances supplémentaires 27 par rapport aux autres portions 7 et 13.

La ou les excroissances 24 représentées à la figure 3 peuvent être avantageusement un ou des bossages 30, de même que la ou les excroissances supplémentaires 27 (non représentées sous cette forme). Ces bossages 30 peuvent être réalisés de toutes les manières techniquement appropriées : pièces rapportées, taillage dans la masse servant à fabriquer la vis 4, moulage, etc.

La ou les excroissances 24 (figure 4) ou/et la ou les excroissances supplémentaires 27 (figure 3) peut/peuvent être formée(s) ou constituée(s) chaque fois par une tige 31 ou un groupe de tiges 31 ou par une lame (non représentée) dressée de préférence au sommet 22 d'un ou des filets 6 de la vis 4 (figure 4) ou respectivement (figure 3) d'une excroissance 24 ou bossage 30 disposé sur un tel sommet 22. Avantageusement, la ou les tiges 31 ou lames sont souples, élastiques, afin de ménager entre autre les objets 2 à transporter.

De telles tiges 31 peuvent consister en des poils, des cordes, des tubes, etc. par exemple en caoutchouc, en matière synthétique, etc.

Les tiges 31 peuvent être fichées et collées dans des trous ménagés par exemple dans le sommet 22 du filet 6. Elles peuvent être orientées dans toute direction nécessaire selon le cas d'espèce, que cette direction soit purement radiale, ou ne coupe pas l'axe 9, que la tige 31 soit inclinée vers l'amont ou vers l'aval, suivant le sens du transport, à partir du sommet 22, inclinée dans le même sens que, ou dans le sens inverse de, la flèche 25, que toutes les tiges 31 aient une même inclinaison par rapport à un rayon correspondant ou qu'au moins une ait un inclinaison différente, etc.

La ou les excroissances 24 agencées de préférence sur le sommet 22 du ou des filets 6 et/ou la ou les excroissances supplémentaires 27 précitées peuvent former, de leur côté également, un filet de vis 32 dont le pas peut être égal à, ou différent de, celui de la vis de transport 4 proprement dite. Cela dépend entre autre du genre de mouvement que l'on souhaite communiquer à l'autre portion 13 ou respectivement à la troisième portion susdite de l'objet 2 à transporter. Ce filet de vis 32 peut être non interrompu (non représenté). Alors, la différence entre les pas des filets 6 et 32 est limitée puisque le filet de vis 32 ne peut normalement pas interférer avec les creux 5 de la vis de transport 4.

Cependant, selon le cas, la ou les excroissances 24 agencées sur le sommet 22 du filet 6 de la vis 4 et/ou la ou les excroissances supplémentaires 27 précitées peuvent former chaque fois un ou des segments 33 de filet de vis 32 séparés alors les uns des autres et dont le pas peut être égal à, ou différent de, celui de la vis 4 proprement dite. Il peut en effet être inutile d'agir, en certains endroits de la vis 4, sur l'autre portion 13 susdite et/ou sur la troisième précitée des objets 2 au cours du transport de ceux-ci.

Le rayon externe ou distance du point le plus éloigné de l'excroissance 24 et/ou de l'excroissance supplémentaire 27, par rapport à l'axe 9 de rotation de la vis de transport 4 proprement dite, peut varier d'une excroissance 24, 27, ou tige 31, à l'autre ou le long de la longueur de l'excroissance 24, 27, considérée lorsque celle-ci est par exemple un bossage 30. Ainsi peut être réalisée par exemple une entrée en contact progressive de l'excroissance 24, 27, et de l'autre portion 13 ou respectivement de la troisième portion d'objet 2 correspondante.

Bien que l'invention a été décrite en faisant référence à plusieurs formes de réalisation particulières de celle-ci, ceux qui sont expérimentés dans le métier sont à même de réaliser différentes modifications à ces formes de réalisation décrites de l'invention, sans sortir de l'esprit ni de la portée réelle de l'invention déterminés par les revendications. Il est entendu que toutes les combinaisons d'éléments qui réalisent sensiblement la même fonction selon sensiblement la même manière pour obtenir le même résultat sont comprises dans la portée des revendications de la présente invention.

Ainsi, le rayon externe du sommet 22 du filet 6 peut être variable le long de l'axe 9 de rotation de la vis 4.

Bien qu'à la base, l'axe non représenté du filet de vis 32 et l'axe 9 de la vis 4 soient superposés, il est concevable qu'ils soient décalés et éventuellement non parallèles.

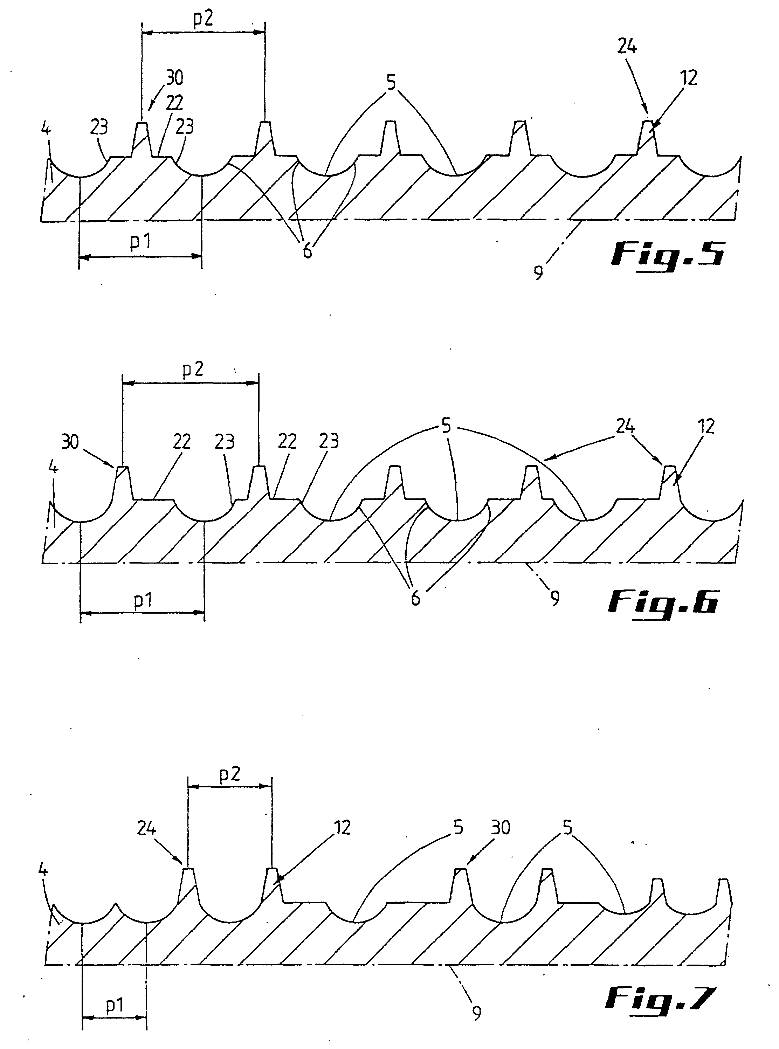

La figure 5 montre un cas où les creux 5 sont identiques en forme et dimensions, de même que le sont les bossages 30. De plus, le pas p1 des creux 5 est fixe comme l'est celui p2 des bossages 30. En outre, les pas p1 et p2 peuvent être égaux.

La figure 6 diffère de la figure 5 par le fait que le pas p2 des bossages 30 varie le long de l'axe 9.

Dans le cas de la figure 7, on peut voir que les dimensions, d'une part, des creux 5 et, d'autre part, des bossages 30 varient de l'un à l'autre. Il en est de même des pas p1 et p2 respectifs.

On comprend que les filets 6 et 32 peuvent être ou non en phase l'un par rapport à l'autre.

Ces variantes représentées dans les figures 5 à 7, prises isolément ou en quelconques combinaisons peuvent être aménagées par l'homme de métier selon qu'il a par exemple besoin d'accélérer ou de ralentir localement la vitesse de transport des objets 2 le long de leur parcours commandé par la vis 4.

Les creux de vis 5 ont été représentés arrondis dans les diverses figures. Il va de soi que ces creux 5 peuvent avoir d'autres formes, telles que carrées, rectangulaires, partiellement hexagonales, etc.

Dans le cas de la seringue 3 comme dans d'autres cas non explicités ci-dessus, le collet 15 (l'autre portion 13), dès qu'il est orienté selon la direction expliquée plus haut et dans la mesure où le guide 8 le permet, peut entrer lui aussi en prise avec les filets 6 qui agissent sur le corps cylindrique 14 (portion déterminée 7), en raison de son autre dimension 18 qui lui permet de tomber entre deux filets 6.

Il va de soi que la vis peut être une vis à une ou à plusieurs entrées.

### Légende des figures

- 1: dispositif de transport
- 2: objet
- 3: seringue
- 4: vis de transport
- 5: creux de 4
- 6: filet de 4
- 7: portion déterminée de 2
- 8: guide
- 9: axe de 4
- 12: moyens pour agir
- 13: autre portion de 2
- 14: corps cylindrique de 3
- 15: collet de 3
- 16: piston de 3
- 17: dimension de 15
- 18: autre dimension de 15
- 19: bords de 15
- 22: sommet de 6
- 23: flanc de 6
- 24: excroissance
- 25: flèche de sens de rotation de 4
- 26: flèche sens d'avancement de 2
- 27: excroissance supplémentaire
- 30: bossage
- 31: tige
- 32: filet de vis
- 33: segment de 32

## Revendications

1. Dispositif de transport (1) d'objets (2) selon une direction et un sens (26) déterminés, comportant une vis de transport (4), dont le creux (5) entre filets (6) successifs est adapté pour entrer en prise avec une portion déterminée (7) des objets (2) à transporter, et comportant un guide (8) agencé sensiblement parallèlement à l'axe (9) de la vis (4) de manière à maintenir dans le creux (5) de la vis (4) ladite portion (7) des objets (2) à transporter,
**caractérisé en ce que** la vis de transport (4) comporte de plus, sur au moins une partie de filet (6) de la vis, des moyens (12) agencés pour agir sur au moins une autre portion (13) des objets (2) au cours de leur transport.

2. Dispositif suivant la revendication 1, **caractérisé en ce que** les moyens (12) agencés pour agir sur au moins une autre portion (13) des objets (2) au cours de leur transport sont situés au sommet (22) de ladite partie de filet (6).

3. Dispositif suivant l'une ou l'autre des revendications 1 et 2, **caractérisé en ce que** les moyens (12) agencés pour agir sur au moins une autre portion (13) des objets (2) au cours de leur transport comportent au moins une excroissance (24) agencée pour entrer en prise temporairement, au cours du transport, avec cette autre portion.

4. Dispositif suivant la revendication 3, **caractérisé en ce que** sur l'excroissance (24) susdite est prévue au moins localement une excroissance supplémentaire (27) agencée pour entrer en prise, au moins temporairement, au cours du transport avec une autre portion de l'objet (2), différente de celles précitées.

5. Dispositif suivant l'une ou l'autre des revendications 3 et 4, **caractérisé en ce que** l'excroissance (24) et/ou l'excroissance supplémentaire (27) est/sont un bossage (30).

6. Dispositif suivant l'une quelconque des revendications 3 à 5, **caractérisé en ce que** l'excroissance (24) susdite ou/et l'excroissance supplémentaire (27) est/sont au moins une tige (31) ou lame dressée au sommet (22) d'un filet (6) de la vis (4) ou respectivement d'une excroissance (24).

7. Dispositif suivant la revendication 6, **caractérisé en ce que** la tige (31) ou lame est souple.

8. Dispositif suivant l'une quelconque des revendications 3 à 7, **caractérisé en ce que** l'excroissance (24) agencée sur le sommet (22) du filet (6) de vis (4) et/ou l'excroissance supplémentaire (27) précitée forme(nt) un filet de vis (32) dont le pas peut être différent de celui de la vis (4) proprement dite

9. Dispositif suivant l'une quelconque des revendications 3 à 7, **caractérisé en ce que** l'excroissance (24) ou les excroissances (24) agencées sur le sommet (22) du filet (6) de vis (4) et/ou la ou les excroissances supplémentaires (27) précitées forme(nt) chaque fois un segment (33) de filet de vis (32) dont le pas peut être différent de celui de la vis (4) proprement dite.

10. Dispositif suivant l'une quelconque des revendications 3 à 9, **caractérisé en ce que** le rayon externe de l'excroissance (24) et/ou de l'excroissance supplémentaire (27), par rapport à l'axe (9) de rotation de la vis de transport (4) proprement dite, peut varier d'une excroissance (24) à l'autre dans le cas de tiges (31) ou le long de la longueur de l'excroissance (24) considérée dans le cas de bossages (30).

## Patentansprüche

1. Vorrichtung (1) zum Transport von Gegenständen (2) in einer vorbestimmten Richtung und Orientierung (26), umfassend eine Transportschraube (4), deren Vertiefung (5) zwischen sukzessiven Schraubengängen (6) angepaßt ist, um einen bestimmten Teil (7) der zu transportierenden Gegenständen (2) aufzunehmen, und umfassend eine Führung (8), die parallel zur Achse (9) der Schraube (4) vorgesehen ist, um in der Vertiefung (5) der Schraube (4) den Teil (7) der zu transportierenden Gegenstände (2) zu halten, **dadurch gekennzeichnet, dass** die Transportschraube (4) weiterhin auf mindestens einem Teil des Schraubengangs (6) Schraubenmittel (12) umfasst, die vorgesehen sind, um auf mindestens einen weiteren Teil (13) der Gegenstände (2) während ihres Transports zu wirken.

2. Vorrichtung nach Anspruch 1, **dadurch gekennzeichnet, dass** die Mittel (12), die vorgesehen sind, um auf mindestens einen weiteren Teil (13) der Gegenstände (2) während ihres Transports zu wirken, sich auf dem oberen Ende (22) des Teils des Schraubengangs (6) befinden.

3. Vorrichtung nach einem der Ansprüche 1 und 2, **dadurch gekennzeichnet, dass** die Mittel (12), die vorgesehen sind, um auf mindestens einen weiteren Teil (13) der Gegenstände (2) während ihres Transports zu wirken, mindestens eine Ausstülpung (24) umfassen, die vorgesehen ist, um temporär während des Transports an diesem weiteren Teil anzugreifen.

4. Vorrichtung nach Anspruch 3, **dadurch gekennzeichnet, dass** auf der Ausstülpung (24) zusätzlich zumindest lokal eine ergänzende Ausstülpung (27) vorgesehen ist, die angeordnet ist, um zumindest temporär während des Transports an einem weiteren Teil der Gegenstände (2) anzugreifen, die von dem vorgenannten Teil verschieden ist.

5. Vorrichtung nach einem der Ansprüche 3 oder 4, **dadurch gekennzeichnet, dass** die Ausstülpung (24) und/oder die ergänzende Ausstülpung (27) Höcker (30) ist/sind.

6. Vorrichtung nach einem der Ansprüche 3 bis 5, **dadurch gekennzeichnet, dass** die Ausstülpung (24) und/oder die ergänzende Ausstülpung (27) mindestens ein Stift (31) oder eine Platte ist, die auf dem oberen Ende (22) eines Schraubengangs (6) der Schraube (4) oder jeweils einer Ausstülpung (24) ausgerichtet sind.

7. Vorrichtung nach Anspruch 6, **dadurch gekennzeichnet, dass** der Stift (31) oder die Platte biegsam sind.

8. Vorrichtung nach einem der Ansprüche 3 bis 7, **dadurch gekennzeichnet, dass** die Ausstülpung (24) auf dem oberen Ende (22) des Schraubengangs (6) der Schraube (4) vorgesehen ist und/oder die ergänzende Ausstülpungen (27) einen Schraubengang (32) bilden, dessen Gang verschieden sein kann von demjenigen der eigentlichen Schraube (4).

9. Vorrichtung nach einem der Ansprüche 3 bis 7, **dadurch gekennzeichnet, dass** die Ausstülpung (24) oder die Ausstülpungen (24), die auf dem oberen Ende (22) des Schraubengangs (6) der Schraube (4) ausgerichtet sind, und/oder die ergänzende oder die ergänzenden Ausstülpungen (27) jeweils ein Segment (33) des Schraubengangs der Schraube (32) bilden, deren Gang verschieden sein kann von demjenigen der eigentlichen Schraube (4).

10. Vorrichtung nach einem der Ansprüche 3 bis 9, **dadurch gekennzeichnet, dass** der Außenradius der Ausstülpung (24) und/oder ergänzenden Ausstülpung (27) im Hinblick auf die Rotationsachse (9) der eigentlichen Transportschraube (4) von einer Ausstülpung (24) zur anderen im Falle von Stiften (31) oder entlang der Länge der Ausstülpung (24) im Fall der Höcker (30) variieren kann.

## Claims

1. Device (1) for transporting objects (2) in a given direction and sense (26), comprising a conveyor screw (4) whose groove (5) between successive threads (6) is adapted to engage with a given portion (7) of the objects (2) to be transported, and comprising a guide (8) which is arranged substantially parallel to the axis (9) of the screw (4) in such a manner as to hold said portion (7) of the objects (2) to be transported in the groove (5) of the screw (4),
**characterised in that** the conveyor screw (4) also comprises, on at least one portion of the thread (6) of the screw, means (12) which are arranged to act on at least one other portion (13) of the objects (2) during their transportation.

2. Device according to claim 1, **characterised in that** the means (12) arranged to act on at least one other portion (13) of the objects (2) during their transportation are located on. the crest (22) of said portion of the thread (6).

3. Device according to either claim 1 or claim 2, **characterised in that** the means (12) arranged to act on at least one other portion (13) of the objects (2) during their transportation comprise at least one protuberance (24) which is arranged to engage temporarily, during transportation, with that other portion.

4. Device according to claim 3, **characterised in that** there is provided on the above-mentioned protuberance (24), at least locally, an additional protuberance (27) which is arranged to engage, at least temporarily, during transportation, with another portion of the object (2), which portion is different from those mentioned above.

5. Device according to either claim 3 or claim 4, **characterised in that** the protuberance (24) and/or the additional protuberance (27) is/are a boss (30).

6. Device according to any one of claims 3 to 5, **characterised in that** the above-mentioned protuberance (24) and/or the additional protuberance (27) is/are at least one rod (31) or lug formed on the crest (22) of a thread (6) of the screw (4) or on the crest of a protuberance (24).

7. Device according to claim 6, **characterised in that** the rod (31) or lug is flexible.

8. Device according to any one of claims 3 to 7, **characterised in that** the protuberance (24) arranged on the crest (22) of the thread (6) of the screw (4) and/or the above-mentioned additional protuberance (27) form(s) a screw thread (32) whose pitch may be different from that of the screw (4) itself.

9. Device according to any one of claims 3 to 7, **characterised in that** the protuberance (24) or the protuberances (24) arranged on the crest (22) of the thread (6) of the screw (4) and/or the above-mentioned additional protuberance(s) (27) each time form(s) a segment (33) of *the* screw thread (32) whose pitch may be different from that of the screw (4) itself.

10. Device according to any one of claims 3 to 9, **characterised in that** the outer radius of the protuberance (24) and/or of the additional protuberance (27), relative to the axis of rotation (9) of the conveyor screw (4) itself, can vary from. one protuberance (24) to another in the case of rods (31) or along the length of the protuberance (24) in question in the case of bosses (30).
